(19) Europäisches Patentamt
European Patent Office
Office européen des brevets

(11) **EP 4 266 985 B1**

(12) **EUROPEAN PATENT SPECIFICATION**

(45) Date of publication and mention
of the grant of the patent:
**15.04.2026 Bulletin 2026/16**

(21) Application number: **21865354.1**

(22) Date of filing: **24.12.2021**

(51) International Patent Classification (IPC):
**A61B 5/00** (2006.01)    **A61B 5/05** (2021.01)
**A61B 5/0507** (2021.01)   **A61B 18/18** (2006.01)
**A61B 5/055** (2006.01)    **A61B 6/03** (2006.01)
**G01R 33/60** (2006.01)

(52) Cooperative Patent Classification (CPC):
**A61B 5/0507; A61B 5/7225;** A61B 5/0017;
A61B 5/4312; A61B 5/7246; A61B 2560/0223;
A61B 2560/0431

(86) International application number:
**PCT/IB2021/062292**

(87) International publication number:
**WO 2022/137207 (30.06.2022 Gazette 2022/26)**

(54) **ELECTROMEDICAL SYSTEM FOR THE NON-INVASIVE DIAGNOSIS OF NEOPLASTIC DISEASES**

ELEKTROMEDIZINISCHES SYSTEM ZUR NICHTINVASIVEN DIAGNOSE NEOPLASTISCHER ERKRANKUNGEN

SYSTÈME ÉLECTROMÉDICAL POUR LE DIAGNOSTIC NON INVASIF DE MALADIES NÉOPLASIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.12.2020 EP 20217275**
**05.02.2021 EP 21155605**
**23.12.2021 IT 202100032537**

(43) Date of publication of application:
**01.11.2023 Bulletin 2023/44**

(73) Proprietor: **Arsmetica Technologies S.r.l.**
**38121 Trento (IT)**

(72) Inventors:
• **BALMA, Massimo**
**38121 Trento (IT)**
• **DALDOSSO, Nicola**
**38121 Trento (IT)**
• **DELOGU, Andrea**
**38121 Trento (IT)**

(74) Representative: **Studio Torta S.p.A.**
**Via Viotti, 9**
**10121 Torino (IT)**

(56) References cited:
US-A1- 2004 254 457    US-A1- 2008 171 949
US-A1- 2012 305 774    US-B1- 9 610 458

• MAIURI MARGHERITA ET AL: "Ultrafast Spectroscopy: State of the Art and Open Challenges", JOURNAL OF THE AMERICAN CHEMICAL SOCIETY, vol. 142, no. 1, 8 January 2020 (2020-01-08), pages 3 - 15, XP055923035, ISSN: 0002-7863, [retrieved on 20220518], DOI: 10.1021/jacs.9b10533

- ZIMMERMAN JACQUELYN W. ET AL: "Targeted treatment of cancer with radiofrequency electromagnetic fields amplitude-modulated at tumor-specific frequencies", ATZHENG -CHINESE JOURNAL OF CANCER, vol. 32, no. 11, 5 November 2013 (2013-11-05), CN, pages 573 - 581, XP055802505, ISSN: 1000-467X, Retrieved from the Internet <URL:https://www.ncbi.nlm. nih.gov/pmc/articles/PMC3845545/pdf/ cjc-32-11-573.pdf> [retrieved on 20220518], DOI: 10.5732/cjc.013.10177
- BACOT VINCENT ET AL: "Phase-conjugate mirror for water waves driven by the Faraday instability", PROCEEDINGS OF THE NATIONAL ACADEMY OF SCIENCES OF THE UNITED STATES OF AMERICA, vol. 116, no. 18, 30 April 2019 (2019-04-30), pages 8809 - 8814, XP002806501, ISSN: 0027-8424
- CLARBRUNO VEDRUCCIO ET AL: "Non invasive radiofrequency diagnostics of cancer. The Bioscanner Trimprob technology and clinical applications", JOURNAL OF PHYSICS: CONFERENCE SERIES, INSTITUTE OF PHYSICS PUBLISHING, BRISTOL, GB, vol. 329, no. 1, 13 December 2011 (2011-12-13), pages 12038, XP020215364, ISSN: 1742-6596, DOI: 10.1088/ 1742-6596/329/1/012038
- YAMAKOSHI Y ET AL: "Experimental Observation of a Nonlinear Characteristic of the Ultrasonic Scattering", IEEE 1986 ULTRASONICS SYMPOSIUM, IEEE, 17 November 1986 (1986-11-17), pages 881 - 884, XP032284858, DOI: 10.1109/ULTSYM.1986.198862
- SATO T ET AL: "Nonlinear parameter tomography system using counterprop agating probe and pump waves", ULTRASONIC IMAGING, DYNAMEDIA INC., SILVER SPRING, MD, US, vol. 7, no. 1, 1 January 1985 (1985-01-01), pages 49 - 59, XP026379848, ISSN: 0161-7346, [retrieved on 19850101]

## Description

## Technical Field of the Invention

The present invention concerns an electromedical system for the non-invasive diagnosis of neoplastic diseases.

## State of the Art

Over the last decades, various technologies and correlated devices have been developed for investigating the properties of biological material.

For example, using computed tomography it is possible to generate a three-dimensional image through successive cross-sectional scans of the body. The majority of activities in the image reconstruction field are based on the use of X-rays and other narrow-beam penetrating radiations, for example gamma rays. The quantity measured and displayed is the absorption of X-rays in each elementary volume within the cross-section of interest.

Emission tomography differs from transmission tomography in that instead of radiating a selected section of the body with penetrating radiations and measuring the quantity discharged from the other side, the penetrating radiation emitted by a special radioactive chemical substance injected into the body is measured and an image of the area through which the emission beam passes is generated. In the case of medical investigations, X-ray tomography presents a health risk due to the effects of the ionizing emissions. Research and development in this field focus on increasingly sensitive sensors to reduce the dose released to the body.

Other image diagnosis methods use non-ionizing radiations. In ultrasound devices, for example, high frequency ultrasonic pulses are transmitted to the body and the images of the shape of the tissues are reconstructed by the reflected pulses (echo data). Although this technique uses non-ionizing radiations, it has some drawbacks: the main one is that the echo data often contain considerable noise and the limited definition is a constraint on its possible applications.

US-A-2008171949 describes a method to analyse tumour tissue using a first electromagnetic signal and a second electromagnetic signal.

Margherita et al., JACS, 2020, 142, 3-15 describes pump&probe technologies as used in biotechnology. CLARBRUNO VEDRUCCIO ET AL ("Non invasive radiofrequency diagnostics of cancer. The Bioscanner Trimprob technology and clinical applications",JOURNAL OF PHYSICS: CONFERENCE SERIES, INSTITUTE OF PHYSICS PUBLISHING, BRISTOL, GB, vol. 329, no. 1, 13 December 2011) describes a diagnostic device to investigate suspected cases of disease and cancer by irradiating a biological tissue with a non- linear radiofrequency oscillator working on 462-465 MHz plus the harmonics while a spectrum analyzer placed in the far field detects by means of a small antenna, the oscillator interaction within the tissue.

A complementary technique for mapping the electric properties of the biological material relates to impedance analysis using weak electric currents or microwave tomography techniques. Despite recent developments, also these techniques suffer from the same drawbacks as the ultrasound methods. The bioelectric mapping methods are mainly based on investigation of the dielectric constant and the conductivity of the biological material.

The conductivity can be obtained from measurements taken at different radio frequencies according to the quantity of imbalance created in a previously stable balanced system by means of a sensing coil. Some diagnostic devices function by measuring the variations of the electric constant and conductivity of the breast tissue. It is also possible to radiate target tissues or materials with electromagnetic radiations and to detect the scattered electromagnetic radiation, using techniques developed mainly for avionics and terrestrial radar systems, to extract the structural characterization of the target (in the time and/or frequency domain).

In the diagnostic field, some devices also make use of the fluorescent radiation stimulated in response to a primary electromagnetic excitation.

Another class of diagnostic techniques refers to systems based on nuclear magnetic resonance (NMR). NMR devices exploit the fact that the nuclei, typically single protons or hydrogen nuclei, have a small nuclear momentum and an associated spin angular momentum. The combined effect of the magnetic and spin momentums gives rise to precessions on the nuclei in the direction of an applied magnetic field. In NMR a magnetic gradient is applied to the sample under examination, where the nuclei tend to align in the direction of the static magnetic field, giving rise to mass magnetization of the sample. A further pulse is then used to perturb the magnetization and the repolarization takes place based on the spin-lattice relaxation time. The precession frequency, known as Larmor frequency, is typically detected between 10 and 100 MHz, and this requires the application of a high intensity magnetic field (0.2-2.5 Tesla). This is the biggest limit of the technique.

Using photon-phonon couplings, when the pulsed EM energy is absorbed by the biological tissue, it is known that part of the impulsive energy is converted into acoustic energy. Some devices have been developed to analyse this information to reconstruct the image of the biological tissue under examination. The absorption is correlated to the permittivity, conductivity and radiofrequency chosen to excite the material under examination.

In the brief and non-exhaustive review described above, all the techniques reported are characterized by the capacity to investigate specific physical responses of the biological target under examination. The physical response is usually obtained and measured at the expense of the increase in entropy of the system under examination. This side effect originates from exposure of the sample under examination to the energy emitted by the detection device (X-rays, static magnetic field, EM field, etc. ...). In some cases a fundamental criticality also emerges: for example in NMR, the additional increase in entropy can reduce or even cancel the capacity to obtain potentially important microscopic information. This is known in cases of nuclear quadrupole resonance, which are cancelled by the high static field of the NMR devices.

Bioelectric properties

The discovery of electric properties in biological tissues dates back to 1926 when it was realised that the dielectric characteristics of tumours are significantly different from those of healthy tissues when radiated by electromagnetic (EM) waves with a frequency around 20 KHz [1]. In particular, and interestingly, Fricke and Morse found that malignant tumours have a fairly high electrical capacity compared to benign lesions or healthy tissues. They reported the polarization effects of these biological systems and noted that the central (oldest) portion of the tumour has a lower electrical capacity compared to the actively growing edge (the capacity decreases with the age of the patient).

Estimation of the parameters of the Fricke-Morse model of the biological tissue is widely used in processing and analysis of bioimpedance data. More recent studies have confirmed that the dielectric properties of cells depend on their type and physiological state: MDA-231 human breast cancer cells, for example, have a mean specific capacity of the plasmatic membrane of 26 $mF/m^2$, in contrast with the value of 11 $mF/m^2$ observed for the T lymphocytes at rest [2].

Without embarking on a lengthy review of the results on this topic, we cite only the recent observation [3] that an EM signal, appropriately modulated, is able to induce self-assembly of the tubulin protein, whereas no effect is observed in spontaneous growth of the microtubules in absence of EM pumping (see the live visualization in [4] where the frequency interval is measured in which the protein mechanically folds and its structure electromagnetically vibrates). Equally interesting is the dielectrophoretic separation of the tumour cells from the blood [2], achieved by applying to the sample a non-homogeneous electric field (with a rotation frequency up to 140 MHz).

Both the above examples illustrate in particular the fact that in biological systems non-thermal effects occur when said systems interact with an applied EM field. A long series of experimental and theoretical studies also leads to the conclusion that cells electrically polarize when they are subjected to an oscillating electric field, which thus induces polarization (dipole) oscillations in biological systems.

It is also reported here, for the sake of awareness of the relevant scientific literature, that the dielectric properties of the cells are determined by the morphology of the cell surface, the dimensions of the cells and other biochemical factors. These effects can be included (within 10% of the real values, with a 90% confidence level) in the specific dielectric model referred to in [2].

Therefore, with appropriate boundary conditions, the problem of a biological disease can be analysed as an anisotropy with respect to a medium (the body) into which an exploratory electromagnetic field is penetrating.

In fact, the cells that form a living tissue communicate by means of gap junction contacts, which allow the passage of ions and other signalling molecules between the cells. This establishes the electric connections between the cells. The biological membranes form a system with high level of molecular integration, in which chemical, lipidic, structural protein and enzymatic components, interacting with one another, combine to form a fundamental and unitary structure.

The cells of the diseased tissues, in particular tumour cells, are characterised by different forms of atypia (metabolic, biochemical and cooperative behaviour), to which alterations of the pathogenic membrane contribute, both at the level of the subcellular vesicles and at the level of the cell surface. The surface is generally rich in electrostatic charges, which determine reciprocal attraction or repulsion: the tissue cells attract one another and remain cohesive due both to the presence of glycoproteic cementing substances and the action of ions neutralizing the surface charges that form a bridge between the cells. All the cells show an electronegative surface. In tumour cells, the electronegativity of the cell surface increases and this is connected to the increase in sialic acid. The electronegativity is not countered by the calcium ions, the importance of which is well known in adhesion. In tumours, the extracellular calcium content diminishes and is not able to facilitate the adhesion.

In addition to the adhesiveness, the fundamental role of the proteins in the cells should be mentioned. In fact, their job is to form and maintain the double lipid layer of the membrane, within which they are free to move by translations or rotations, according to the fluidity of the lipid layer. This property is markedly altered in the case of tumours.

Finding new targets for the study and understanding of tumour cells is one of the biggest challenges in current medical research. Specifically, predicting whether a tumour is inclined to progression or remission is a very important priority. For this purpose, various approaches have been attempted to anticipate the behaviour of the tumour cells before and after the beginning of treatment.

Another important point to consider is that, undoubtedly, during the progression of the tumour, changes occur not only in the macroscopic structure and viscoelasticity of the cells, but also in their internal microscopic dynamics. This happens because the alterations in cell morphology and packing naturally influence the pathways through which the organelles, the water and other biomolecules can move. Curiously, despite the consolidation of this idea and the fact that the cells contain mainly water molecules, very few studies have focused on the properties of the intra- and extracellular water, relating them to the behaviour of the tumour cells.

Given the nature of the cell medium, the modifications in the characteristics and properties of the water can be correlated with single or distinct movement types and this differentiation may be the key to explaining how and why water dynamics change within cells with different prognoses [5]. The possible differences can be traced back to the different ways in which the water can interact within the complex cell environment. While the weakly interacting water molecules show a behaviour similar to free water, scattering at 37°C with a scattering coefficient of approximately $3 \times 10^{-9}$ m$^2$/s, the water molecules confined, for example, by folded proteins, cell membranes and organelles, show different properties since their mobility and therefore effective scattering is limited [6].

The variation in the scattering coefficient can influence, also significantly, the response of the tissues when exposed to an electromagnetic field, explaining how the phenomenon of electromagnetic interaction differs as the cell prognosis differs, and can therefore represent a new vehicle for specific information transduction.

In general, different events are known at cell membrane level, such as:

- loss of inhibition due to the contact;
- reduction in adhesiveness;
- increase in mobility;
- increase in the presence of bound water.

These phenomena lead to the conclusion that the onset of disease causes new degrees of freedom in terms of the electronic and ionic charges involved in the cell exchange dynamics.

Furthermore, the detachment of the cells from the tumour and the association of the tumour cells with the bone are strongly influenced by the cell-cell cohesion and the cell-substrate adhesion, attributes that give the tumours quantifiable biophysical properties. These properties are the elasticity of the tumour (a measurement of the capacity of a tumour to deform in response to an applied stress), the viscosity of the tumour (an indication of the cell motility within the tumour) and the compactness of the tumour (a dynamic and complex manifestation of the cell-cell cohesion and cell-substrate adhesion). These phenomena are essential for investigating the biophysical and biomolecular changes that accompany the malignant progression of the tumour.

Various adhesion factors have been studied as markers for the presence of cancer.

By way of example, hyaluronic acid (HA), a glycosaminoglycan, regulates cell adhesion and migration. Hyaluronidase (HAase), an endoglycosidase, breaks down the HA into small angiogenic fragments. Using an assay similar to the enzyme-linked immunosorbent assay, an increase in HA levels (3-8 times) was found in prostate cancer tissues (CaP) compared to normal tissues (NAP) and benign tissues (BPH) [7]. The majority (75-80%) of HA in prostate tissues was found in the free form. The primary CaP fibroblasts and the epithelial cells secrete 3-8 times more HA than the respective NAP and BP cultures. Only the CaP epithelial cells and the established CaP lines secreted HAasi and the secretion increased with the tumour stage and the metastases.

A stromal and epithelial pattern of expression of HA and HYAL1 was observed in the CaP tissues. While the high HA colouring was observed in the stroma associated with the tumour, the HYAL1 colouring in the tumour cells increased with the tumour stage and the metastases.

While HA with higher or intermediate molecular weight was found in all the tissues, the HA fragments were found only in the CaP tissues. In particular, the high-quality CaP tissues, that showed high levels of HA and HYAL1, contained fragments of angiogenic HA. The stromal-epithelial expression of HA and HYAL1 can promote angiogenesis in CaP and can serve as an electrically charged vector for the electromagnetic interaction.

Obviously the greater freedom of movement of these available charges, typical in a pathological condition (rotary motion, ionic currents), can be used to investigate the biophysical state of the tissues by means of an electromagnetic interaction.

When irradiated by an electromagnetic field, the molecules such as the water and the proteins aim to form a line along the polarization of the field, to minimize the potential energy of the dipoles.

From the spectroscopic analysis, the rotary motion of the water molecules bound to macro-molecules has resonance in frequencies between 100 and 1000 MHz and could be used to provide a signal with high information content. The technological problem is its transduction since it has an extremely weak intensity.

While conventional biology is based mainly on the microstructure of the biological material, namely the cellular or molecular structure as in molecular biology or microbiology offered by physical-chemical concepts, biological material has recently been recognized as an electromagnetic aggregate and a complex electric network system. Therefore, a variety of new mechanical, electromagnetic and electrodynamic effects are described in the latest literature also in cumulative terms; not only for the electrical properties and functioning of the biological material itself, but also for the interactions of the external electromagnetic fields with the biological material as a coherent whole considering cooperative effects.

In the physical world, the structure and properties of material can be described in two ways: description of particles (atom, molecules and/or elementary particles) for microstructures and continuum (medium or fluid) for macrostructures. In the same way, the structure and properties of biological material can also be described in two ways: descriptions with cells, molecules and elementary "Biologically Closed Electric Circuits" for the microstructures and description of the continuum (fluid) for the macrostructures.

## Object and Summary of the Invention

A need is therefore felt in the sector for non-invasive diagnoses to determine neoplastic diseases.

The object of the present invention is to provide an electromedical system for the non-invasive diagnosis of neoplastic diseases.

According to the present invention, an electromedical system is provided as claimed in the attached claims.

## Brief Description of the Drawings

Figure 1 shows a simplified view of the synchronization field and biological oscillators.

Figure 2 illustrates a competition between slow external oscillations (frequency W and force F0) and rapid internal oscillations (frequency $\omega 0$) when $W << \omega 0$. According to F. Kaiser [9], if $W << \omega 0$ and F0 grow in the direction abcdef, the internal oscillation of the radiated system is progressively obliged to reduce its frequency until it synchronizes with the external frequency. The phases b-c-d-e represent transitory moments of oscillatory disarray.

Figure 3 illustrates the case (graph A) in which the frequency of the external oscillation is almost equal to that of the internal system ($\lambda \approx \omega_0$); after a certain external radiation time an amplitude jump of the internal oscillation is obtained by resonance, conserving the same frequency. If $\lambda << \omega_0$, after a certain time the system oscillates with frequency $\lambda$ and lower amplitude (graph B).

Figure 4 illustrates the phenomenon of the phase-conjugate mirror; to obtain the effect described, two waves coming from opposite directions, called pump waves, are radiated on the non-linear medium. When a third (probe) signal wave is transmitted on the medium, it produces the counter-propagating fourth wave (idler) time-reversed with respect to the signal.

Figure 5 illustrates a block diagram of the electromedical system.

Figure 6 schematically illustrates a functional configuration of the beams of a source unit of the electromedical system of Figure 5.

Figure 7 schematically illustrates that the formation of the parametric instability depends on the state of the tissue disarray.

Figures 8 and 9 illustrate the transduction of the state of homeostasis, as detected by a receiving unit of the system of

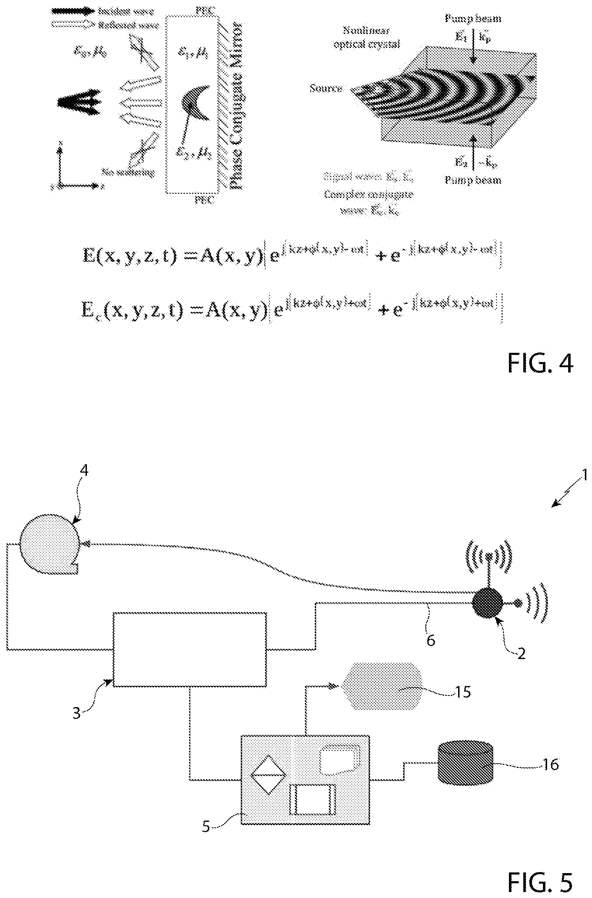

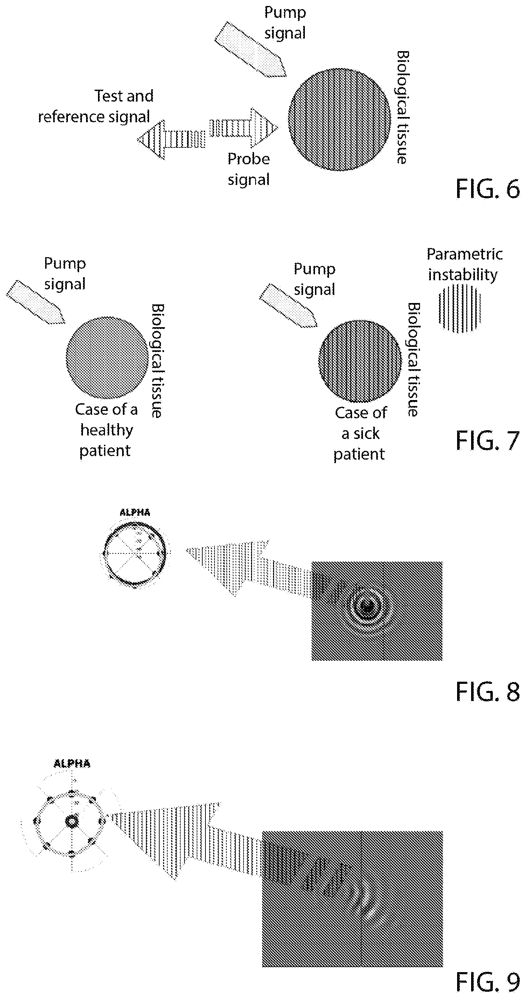

Figure 5 by means of the field map received, to an antenna of the system of Figure 5 in the case of a healthy tissue (Figure 8) and in the case of a diseased tissue (Figure 9).

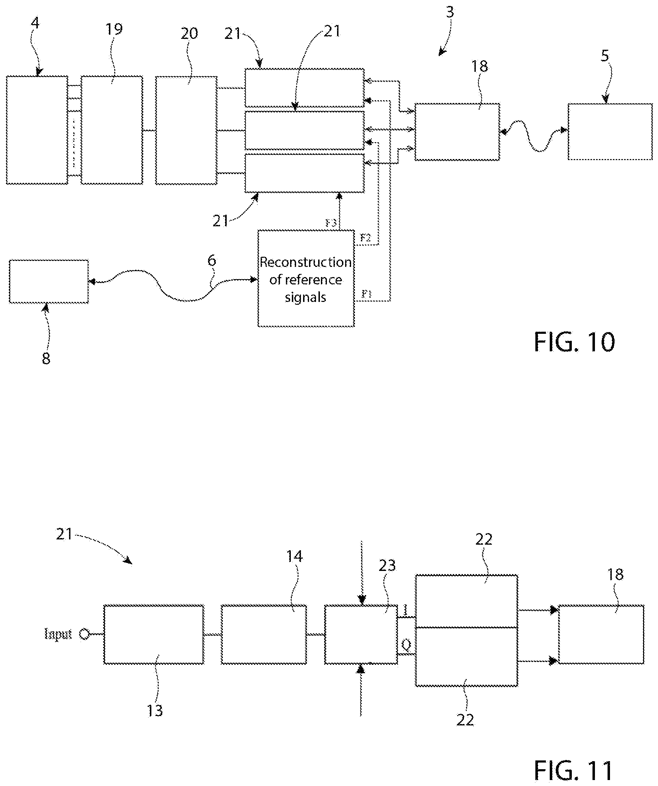

Figure 10 illustrates a circuit block diagram of a receiving unit of the electromedical system of Figure 5.

Figure 11 illustrates an architecture of the reception chain on the single band.

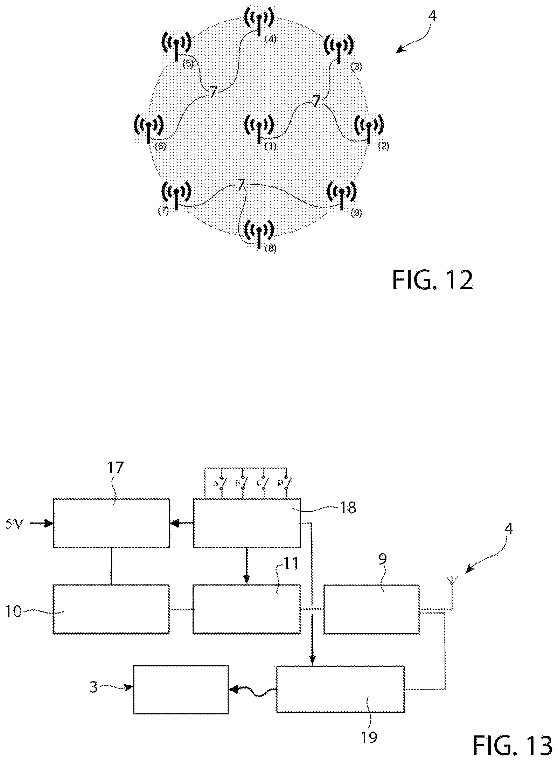

Figure 12 schematically illustrates the configuration of the antenna array.

Figure 13 schematically shows a circuit block diagram of the probe of the electromedical system of Figure 5.

## Description of Preferred Embodiments of the Invention

The present invention will now be described in detail with reference to the attached figures to allow a person skilled in the art to produce it and use it. Various modifications to the embodiments described will be immediately evident to the persons skilled in the art and the generic principles described can be applied to other embodiments and applications without thereby departing from the scope of the present invention, as defined in the attached claims. Therefore, the present invention must not be considered limited to the embodiments described and illustrated but must be given the widest scope in accordance with the characteristics described and claimed.

Where not defined otherwise, all the technical and scientific terms used here have the same meaning commonly used by persons of ordinary experience in the field pertaining to the present invention. In the case of conflict, the present description, including the definitions provided, will be binding. Furthermore, the examples are provided for purely illustrative purposes and as such must not be considered limiting.

In particular, the block diagrams included in the figures attached and described below are not be understood as representations of the structural characteristics, namely constructional limitations, but must be interpreted as a representation of functional characteristics, that is, intrinsic properties of the devices and defined by the effects obtained, namely functional limitations and which can be implemented in various ways, therefore such as to protect the functionalities of the same (possibility of functioning).

In order to facilitate understanding of the embodiments described here, reference will be made to some specific embodiments and a specific language will be used to describe the same. The terminology used in the present document has the purpose of describing only particular embodiments and is not intended to limit the scope of the present invention.

Synchronization

The new extraction method subject of the present description is aimed at obtaining, by non-invasive electromagnetic means and without the use of ionizing radiations, information useful for understanding and analysis of the state of the biological tissues and refers, considering the above introductory premise, also, but not only, to the phenomenon of "synchronization".

The history of synchronization dates back to the 17th century when the Dutch scientist Christian Huygens reported his observations on the behaviour of two pendulum clocks which he had just invented, which synchronized with each other by means of a vibrational mechanical coupling. This invention significantly increased timekeeping accuracy and helped him to tackle the problem of longitude determination.

In the mid-19th century, in his treatise 'The Theory of Sound", Lord Rayleigh described an interesting phenomenon of synchronization of acoustic systems: 'When two organ pipes of the same pitch stand side by side, complications arise which frequently create problems in practice. In an extreme case, the pipes can reduce each other to silence. Even when the reciprocal influence is moderate, it may still go so far as to cause the pipes to play in absolute unison in spite of inevitable small differences". Therefore, Rayleigh observed not only mutual synchronization when two distinct but similar pipes begin to play in unison, but also the correlated effect of damping of the oscillation when the coupling causes suppression of the oscillations of interacting systems.

In many natural situations, interactions are present between several oscillators. If two oscillators (whether mechanical or otherwise) can modify their rhythms, a large number of systems can also do so.

Apart from the possible variety of dynamic systems and coupling geometries between oscillators, an essential aspect of the overall behaviour of a group of interacting oscillators is the transition from a disorderly state to a situation of synchronization (more orderly state), when the intensity of the coupling between the systems exceeds the critical threshold. The Kuramoto model [8] constitutes a simplified description of the dynamics of a group of interacting oscillators, where the mechanism of birth and maintenance of the synchronization is described.

In its original version, it presents as a population of N oscillators with phase $\theta_i(t)$ having natural frequencies $\omega_i$ distributed according to a probability density $g(\omega)$, and coupled proportionally to the sine of the phase differences:

$$\theta_i = \omega_i + \frac{\cdot}{N} \cdot \sum_{j=1}^{j} \sin(\theta_j - \theta_i)$$

The factor $1/N$ guarantees good behaviour of the model in the thermodynamic limit $N \to \infty$, while $K$ represents the coupling intensity. Clearly, for $K = 0$ the oscillators evolve each according to its natural frequency, $\omega_i$.

The equation therefore describes the dynamics of the population of oscillators coupled on any topology. It is used, for example, as a model of the dynamics of electrical distribution networks, or of biological systems interacting at various levels.

The present invention is based on the availability of an electronic architecture in which an active oscillator, agile in frequency in its operating band (i.e. free), acts as an active stimulus relative to the more or less complex group of oscillators that form an integral part of the biological material to be examined. The operating band of the active oscillator shall be commensurate with the specific characteristic frequencies $\omega_i$ of the oscillators to be synchronized. The coupling vector between the active oscillator and the interacting oscillators consists of the electromagnetic field of stimulus.

One of the most critical aspects for correct functioning of the method and the device is the use of both:

(i) corresponding frequency bands between the stimulus oscillator frequencies and the biological oscillator frequencies,
(ii) coupling factor $K$, which can be experimentally calibrated, thus making the synchronization phenomenon transducible into the interferometric modes described below.

It is also evident that if the coupling vector is to be the electromagnetic field, the oscillating elements of the network must have prerogatives such as to interact with the stimulus field of the active oscillator. In fact, the field will act mainly on ionic charges and dipole moments belonging to the biomolecular structures.

The active source must be free to interact with the elementary constituents of the biological medium to create a specific coupling pattern, linked to the principle of minimization of the energy of the global eigenstate that describes the coupled system formed of the active external source and the passive network of the oscillators.

We will see below that during the stimulus (pump) conditions, the oscillating material will behave in a very particular manner relative to the probe field.

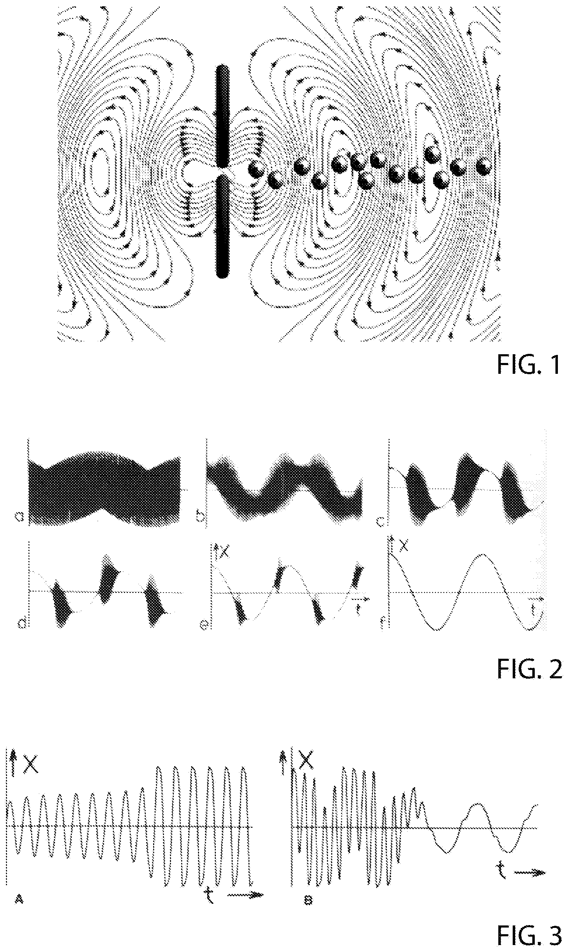

The description in frequency of the collective behaviour of the biological oscillators (see also Figure 1), synchronized by the stimulus field, is illustrated by the theories of F. Kaiser [9]. It is hypothetically defined that the collective motion of the tissue has an oscillation frequency of $\omega_0$. In the interaction models between non-ionizing radiations and living material, Kaiser considers the competitive interactions that can occur between low frequency external EM oscillations (slow oscillations) and high frequency internal oscillations (rapid oscillations) of the radiated biological system, and the interactions dominated by the external oscillation frequency W, the internal oscillation frequency $\omega_0$ and the force $F_0$ of the external oscillation (see Figure 2).

It is shown that when an internal oscillation with frequency $\omega_0$ is perturbed by an external oscillation with force $F_0$ and frequency W much lower than $\omega_0$, with the increase in $F_0$ the internal oscillation undergoes a series of quasi-periodic and irregular or non-periodic states until it is completely synchronized. At that moment, the internal oscillation has synchronized its original frequency with the new frequency W set from the outside and oscillates with a fixed amplitude $F_0$. The phenomenon depends heavily on W and $F_0$.

It should be noted that during the time t necessary for complete synchronization of the internal oscillation with the new values of W and $F_0$ set externally, there are moments in which the internal oscillatory phenomenon reaches quasi-periodic and non-periodic states. At the end of the synchronization process, when the internal oscillation has settled in frequency and intensity on the externally set values, a new oscillatory order is reached, in the sense that the molecular process on which the external radiation has acted continues in an orderly manner, but with new frequency and intensity values (see example in Figure 2).

In the models of F. Kaiser there are other interactive possibilities between external and internal oscillations (graph A of Figure 3). If the frequency W of the external oscillation is equal to the frequency $\omega_0$ of the internal oscillatory process, after a certain external stimulation time there will be an amplitude jump in the internal oscillations which, however, maintain the same frequency (resonance excitation). If, on the other hand, the frequency W is much lower than the frequency $\omega_0$, after a time t there will be a jump to oscillations with lower amplitude and frequency W (graph B of Figure 3).

### The operating principle: biological phase conjugation

The non-linear and parametric processes are central in the wave propagation mode in complex environments. They are at the centre of numerous applications in high frequency optics and acoustics.

In the case of optics, the possibility of generating a phase conjugation through processes such as four-wave mixing, so as to create a "phase-conjugate mirror" (see Figure 4) is well known. When a monochromatic source point emits a wave through the optically nonlinear crystal, the phase-conjugate mirror generates a counter-propagating wave, which refocuses in the same position as the source. This behaviour demonstrates how the crystal behaves like a time reversal device in these conditions.

It is important to note that the effect of the pump waves is equivalent to a time modulation of the refractive index with frequency double that of the signals used.

Recently the possibility of obtaining the phase conjugation on acoustic waves in a liquid through the induction of Faraday instability [10] has been discovered and demonstrated. In this case, the pumping occurs mechanically with one single vibrational source, which shakes the liquid so as to compete with the force of gravity.

This document discloses the capacity of the biological media to behave like phase-conjugate mirrors, when subjected to an electromagnetic pump stimulus.

In the extracellular spaces, there is a large amount of water in which many ions are dissolved. When the frequency of the external pumping field causes a charge anisotropy (separation of positive ions from negative ions), caused by the fact that the different signs are accelerated in opposite directions, intense local electrostatic forces are generated that try to somehow reabsorb the spatial anisotropy.

The high frequency oscillations, in cases where the separation between positive ions and negative ions is no longer negligible, induce in the biological medium a space-time modulation of the local permittivity, similar to a plasma oscillation.

In the case of the present invention, an electromedical system 1 for the non-invasive diagnosis of neoplastic diseases is used to activate the phase conjugation in the biological medium (namely the patient).

The steps to activate the phase conjugation in the biological medium are therefore the following:

a) the electromagnetic pump signal is activated, in particular by means of an oscillator with frequency agility, which can vary in discrete sub-bands in the range from 300 MHz to 2800 MHz such as, for example, but not necessarily, from 900 MHz to 960 MHz, from 1800 MHz to 1920 MHz. The electromagnetic pump signal acts as an activation drive stimulus for the passive biological oscillators (charges). The coupling vector is the electromagnetic field, mainly on the longitudinal field component, by means of the radiation pressure connected to the latter. The stimulus frequency must be near (typically, but not necessarily, 20% of the nominal frequency value) that of the possible oscillation of the biological oscillators;

b) after a preliminary stimulus period, which can vary from a few seconds to thirty seconds, the elementary oscillators of the biological medium enter a resonant, synchronized and coherent state;

c) the synchronized and coherent structure of the medium presents a space-time modulation of the dielectric permittivity, with time period equal to double the stimulus period.

The electromagnetic pump signal constitutes, in the present invention, the parametric excitation of the biological tissue which is comparable to a state of Faraday instability. If the tissue is outside its state of homeostasis, the periodic oscillation of the stimulus induces its coherent destabilization, like the Faraday instability for a liquid interface (see for example Figure 7). This instability can be detected by making it interact with a second electromagnetic probe signal striking the biological medium.

If the frequency of the electromagnetic probe signal is equal to the modulation frequency of the dielectric permeability, which is equal to half the frequency of the electromagnetic pump signal, then the interaction of the signals involved gives rise to an idler beam in response, reversed in time and in the wave vector.

We have therefore disclosed that the biological material in a disordered state, therefore far from homeostasis, synchronized by an electromagnetic pump signal, acts like a phase-conjugate mirror relative to a second incident beam, giving rise to a third response beam reversed in time and in the propagation direction.

The response waves refocus on any initial emission source, thus allowing, according to an inverse and non-invasive procedure, external evaluation and measurement of a specific property of the material under examination, namely that of being in a state of non-homeostasis.

The method and the system

It is well known that tumour cell membranes have different electrochemical properties and a different distribution of the electric charges compared to normal tissues [11]. The homeostasis of tissues in which a tumour neoformation is present is completely lost, and as such it is possible to analyse it by means of the method (not claimed) and the electromedical system 1 of the present invention.

Figures 7, 8 and 9 schematically illustrate the different results obtained during application of the method and the electromedical system 1 according to the present invention.

Among the many variations compared to healthy cells, tumour cells have lower potassium concentrations and sodium and (above all) water content higher than normal cells [12-14]. Consequently, cancer cells show a greater permittivity, interacting differently from normal cells when an external EM field is applied. This characteristic is exploited by conventional imaging systems that use exploratory electromagnetic fields (microwave tomography).

The present invention does not use the principles of radio-wave tomography.

The electromedical system 1 for applying the method described above consists essentially of an electromagnetic source unit 2, a receiving unit 3, a multi-band and multi-channel antenna 4 and a software installed on a computer for processing (processing unit 5) the data, appropriately connected to one another (see Figure 5).

In other words, an electromedical system 1 for the non-invasive diagnosis of neoplastic diseases is described which comprises the electromagnetic source unit 2, the receiving unit 3, the multi-band and multi-channel antenna 4 and the processing unit 5 provided with a data processing software.

The electromagnetic source unit 2 forms the active generation and transmission part of the electromedical system 1. The electromagnetic source unit 2 is configured to generate and radiate the electromagnetic pump, probe and test and reference signals.

In particular, according to the appropriate configurations, the signals can be emitted by physically separate radio frequency antennae and generators or the signal radiating and/or generating parts can be shared. The electromagnetic source unit 2 gives rise to several spectral and spatial field components, which have specific roles and functions. In terms of radiation characteristics, the near field components act in an essentially reactive manner with the oscillators of the biological network and act as a pump stimulus. The radiative field (probe field) components, interacting with the material subjected to pumping, interact electromagnetically with the oscillating material, set in coherent motion by interaction with the electromagnetic pump signal.

In particular, the electromagnetic source unit 2 can comprise a pump antenna and a probe antenna to emit the electromagnetic pump signal and the electromagnetic probe signal respectively. Any modulations can be superimposed on the radiated signals, normally in continuous wave (CW), in order to implement adaptive techniques in the search for the best pump synchronism frequency. The frequency of the electromagnetic pump signal can vary in discrete sub-bands in the range from 300 MHz to 2800 MHz. The frequencies of the probe and the test and reference electromagnetic signal will be consequently bound to the above bands, since the probe frequency is equal to half the pump frequency.

To simultaneously and effectively obtain scanning on several bands, harmonic generators (of the electromagnetic source unit 2) can be used, in which the various fundamental and harmonic bands can have a pump and probe role, also interchangeable.

In other words, the electromagnetic source unit 2 can comprise harmonic generators to generate the electromagnetic pump signal and the electromagnetic probe signal.

The signal generation must guarantee a frequency agility, which is a function of the separation in frequency between pump source and biological oscillator. Typically the value of 20% with respect to the nominal frequency value is required, so as to tune in and synchronise with the frequencies of the passive biological oscillators. This frequency agility can be obtained by means of free oscillators, or by means of scans in frequency controlled through analog and/or digital techniques.

The amplitude of the signals transmitted in the area of contact with the biological tissue lies in the range between 2 V/m and 20 V/m. The amplitude of the level can be used as a synchronization parameter. The synchronization is linked to non-linear phenomena.

In further detail, the electromagnetic source unit 2 comprises antennae to emit the electromagnetic pump signal and the electromagnetic probe signal.

Preferably, the antennae of the electromagnetic source unit 2 have an impedance matching in the bands of use to allow a correct level of radiation, equal to a return loss of -20 dB.

The receiving unit 3 is configured to receive and measure in amplitude and phase the signals generated by the electromagnetic source unit 2, captured by the multi-band and multi-channel antenna 4.

The multi-band and multi-channel antenna 4 operates as a spatial and spectral filter by means of its radiant configuration of an array of independent and decoupled elements, appropriately distributed in space, such as to map the field distribution received.

A fibre optic link 6 (of the electromedical system 1) connects the electromagnetic source unit 2 to a local oscillator of the receiving unit 3, thus allowing coherent field reception in terms of amplitude and phase. Command and control signals also travel on the optical fibre 6 between the receiving unit 3 and the electromagnetic source unit 2.

The receiving unit 3 must allow reception of the signals correlating them in space and time, and the demodulation of said signals if necessary. Modulation of the test and reference signal can be deliberately imparted by the electromagnetic source unit 2, but could also be linked to phenomena such as microdoppler phenomena superimposed on the test and reference signal, and suggesting oscillatory dynamics within the biological medium, carriers of information on the state of homeostatic order or disorder of the tissues. Analysis of the superimposed modulations is considered important for any identification of the main biomolecular aggregates (proteins, bound water, ions, etc.) composing the synchronised biological oscillators.

The multi-channel and multi-frequency reception must take place in real time and simultaneously on all the channels or, if switching systems are used, the switching must be sufficiently rapid to allow spatial mapping of the signals in phase and amplitude, such that the latency time for the reading is negligible with respect to the physical variations of the signal to be received. Consequently, the switchings and readings of the signals must be carried out with an overall period of less than 0.04 seconds for each measurement frame.

The computer (the processing unit 5), by means of the software, allows the amplitudes of the signals measured by the receiving unit 3 to be displayed on a screen in real time.

The diagnosis of the state of homeostasis is carried out based on the signals which, by means of the biological phase conjugation mechanism, demonstrate the presence of said state by measurement of the interference detected. Appropriate selection rules and algorithms that combine the analysis of the various signals are fundamental for discriminating the significant thresholds and interference levels. This concept is schematically illustrated also in Figures 8 and 9.

The logic of the electromedical system 1 is based on the interfering electromagnetic interaction between test and reference signal and the phase-conjugate signal, obtained from interaction of the electromagnetic probe signal with the collective and cooperative type effects induced by the electromagnetic pump signal.

The coherent radiation beam coming from the transmitter of the present electromedical system 1 locally excites the dipole resonant oscillations so as to convey the transport of energy in the biological system.

In the case of tumour metabolism, a transition of the system occurs to a state of parametric Faraday instability (see also Figure 7) due to the non-linear coupling of dipolar vibrations with elastic vibrations of DNA, proteins and membranes in a state of non-homeostasis relative to the pump signal.

Under the action of the radiation of the invention, the guided parametric instability behaves like a biological phase-conjugate mirror, and its activation is identified and quantified by means of the mapping of the field received from the antenna 4 (see also Figure 7). Diagnosis of the altered biological state, also defining diagnostic and prognostic differentiation levels of said state, is therefore a simple and immediate process by means of a correlation phase between the disease and the field distribution detected.

The receiving unit 3 is connected to the array of independent antennae which, with their arrangement, allow analytical reconstruction of the incident wavefront, coming from the interaction of the fields of the electromedical source unit 2 with the biological tissues. A holographic algorithm allows the qualitative information on the state of health of the tissues to be presented graphically on a display 15 and in real time; said information can then be quantified by means of a subsequent post processing phase, correlating on the database of the experimental data already stored. The machine learning and classification phases are part of the post-processing subsystem.

By way of non-limiting application example of the method and system, we describe the procedures according to the invention for the extraction of information on the homeostasis of prostate tissues.

The examination is non-invasive, and as such can be performed directly on the patient without the risk of side effects. The patient stands in front of the array of antennae 4, at a distance of between 110 and 160 centimetres from them. Once all the reception and transmission devices relative to the electromagnetic source unit 2 and the receiving unit 3 have been connected and activated, as in the configuration of Figure 5, the pump antenna (of the electromagnetic source unit 2) radiating the electromagnetic pump signal is placed near the prostate organ. The pump antenna must be positioned in the area of anatomical windows, as used for ultrasound investigations, through which the stimulus signal can reach the tissues of the organ under examination. In the case of the prostate, the anatomical windows that can be used are the perineum and the suprapubic area. The source (the antenna) which gives rise to the electromagnetic probe signal is also positioned in the same anatomical windows. The electromagnetic probe signal interacts with the tissues according to what is described in the previous paragraphs. The array of antennae 4 receives the interfering signals, as in the diagram of Figures 5 and 12. The organ is explored by moving the sources (the probe 8), kept as far as possible in contact with the epidermis, albeit through tight clothing, roto-translating said sources with respect to the axis of the body.

The representation of the wavefront received from the array represents a measurement of the phase conjugation level obtained within the tissues stimulated.

The system

The electromedical system 1 for the non-invasive diagnosis of neoplastic diseases is essentially composed, as already described, of the electromagnetic source unit 2, the receiving unit 3, the multi-band and multi-channel antenna and the software installed on a computer for data processing (processing unit 5), appropriately connected and interacting with one another.

The electromagnetic source unit 2 forms the active generation and transmission part of the system: from this unit the pump, probe and test and reference signals are generated and radiated.

In the present implementation, the electromagnetic source unit 2 is contained in a handpiece (probe 8), so that it can be held in the hand and used for scanning the area under examination. In particular, the probe 8 is configured to be placed in contact with the area of the patient under examination during the scan.

The electromagnetic source unit 2 includes a free oscillator 9 able to generate an RF signal at fundamental frequency, at a UHF band frequency, and its harmonics. The handpiece (the probe 8) also contains the power-supply circuits (with relative high-capacity lithium batteries 10) and control circuits (for example, to monitor correct operation of the oscillator and the battery charge status) in addition to a high-speed fibre optic transmitter 19, which takes a part of the signal of the oscillator 9 and transmits it in optical fibre 6. The probe 8 also includes circuits for management of the lithium battery charge using an external power-supply 11 at 5V (for example, a standard USB). In particular, the probe 8 also comprises a battery charger 17.

On the handpiece (on the probe 8) there are four control buttons 12: A, B, C, D. The pressing of a key must be notified together with information on which key has been pressed; for this purpose the line enabling the optical carrier on the fibre will be used. The "key pressed" event and the "key number" information is transmitted by a codified modulation of the optical signal. This modulated signal can also transmit other information on the operating status of the probe 8 and the battery charge level.

The receiving unit 3 includes three receiving channels 21 to simultaneously receive the signal transmitted by the probe 8 at the fundamental frequency and at the second and third harmonic. Each receiving channel 21 includes an amplifier 13, a radiofrequency filter 14 and an I/Q mixer, with low-pass filter in output, to receive the component in phase and in quadrature of the signal received with respect to the one transmitted, and therefore allow amplitude and phase measurement.

The local oscillator signal is generated for each of the receiving channels 21 starting from the test and reference signal received via optical fibre 6 from the probe 8, by means of appropriate amplifiers 13 and filters 14 (fundamental frequency F1) and also multipliers x2 (frequency F2) and x3 (frequency F3). In this way the signal received and the local oscillator signal are intrinsically at the same frequency and the output of the I/Q mixer is represented by a direct voltage proportional to the amplitude of the signal received, multiplied respectively by the sine and the cosine of the phase difference between signal received and reference signal.

Any band disturbances present, also at a frequency very near the measurement frequency, cause an oscillating signal at output of the I/Q mixer and can be eliminated by means of a low-pass low-frequency filtering stage.

The antenna 4 comprises dipole elements 7 (indicatively, but not exclusively, 9, 32, 64 or 128 elements), receiving in broadband and having reduced dimensions (indicatively, but not exclusively, a few centimetres) and allows measurement of the electromagnetic field generated by the probe 8 which interacts with the district or organ of the patient under examination at different points on a surface positioned at a given distance, indicatively between 1 m and 2 m.

The receiving elements 7 of the antenna 4 can be passive broadband elements (for example, biconical dipoles or Vivaldi antennae) or elements resonating at one of the frequencies received, typically the probe frequency (for example, inductively or capacitively charged dipoles). These elements are decoupled from the signal cable by means, for example, of ferrite balun.

The receiving elements 7 of the antenna 4 can also be composed of active elements (such as, for example, very small dipoles with respect to the wavelength with an integrated broadband amplifier). In this case it is possible to achieve adequate measurement sensitivity with very small receiving elements and therefore measure the signal received on the measurement surface with a reduced spacing between the measurement points (for example, from 1/5 to 1/10 wavelength).

With particular reference to Figure 10, the signal received from each single element 7 of the antenna 4 is sent to the receiving unit 3 by means of a high-insulation radiofrequency switch device 19 (indicatively at least 80 dB). This high-insulation switch device 19 can be composed of a switch and one or more minor insulation circuit breakers (for example, 40 dB each) to obtain the required insulation level. The switches and circuit breakers must be composed of PIN diodes, MOSFET switches or equivalent systems to obtain reduced switching times (indicatively in the order of 1 $\mu$s).

The input switch (switch device 19) also allows insulation of the input of the receiving unit 3 from the antennae 4 and also connection of it to an internal signal generator to perform zero-setting and calibration of the I/Q mixers of the three receiving channels 21.

The broadband signal coming from the selected antenna 4 is then separated into the three main components (fundamental, second and third harmonic) by means of a separator filter 20 (diplexer) (of the receiving unit 3) and sent to the three receiving channels 21.

With particular reference to Figure 11, a controller 18 of the receiving unit 3 controls the input switches and circuit breakers and reads the amplitudes of the signals I (in phase) and Q (counterphase) by means of appropriate separate A/D converters 22 with low conversion time (indicatively 1 $\mu$s per sample or less). In order to optimize the acquisition speed and the insulation between the receiving channels 21, two A/D converters 22 are used for each receiving channel 21 (to simultaneously read the signals I and Q 23).

Furthermore, in order to improve the sensitivity of the receiving unit 3 it is also possible to take averages between several successive measurements. Indicatively, at the A/D conversion speed indicated above, 256 averages per antenna 4 and receiving channel 21 allow more than 20 measurements per second to be taken on an antenna 4 complete with 128 receiving elements 7.

Furthermore, the controller 18 also periodically takes the zero-setting and calibration measurement of the receiving channels 21, measurement of the frequency of the signal received from the probe 8 and measurement of the internal temperature of the I/Q mixers (for any correction of their temperature response).

All the data measured and in particular the data of the signals read for each of the elements of the antenna 4 and for the three frequency bands are then transmitted to the computer (processing unit 5) for the subsequent processing operations, presentation and storage of the results (in a memory 16). The data are transmitted to the computer (processing unit 5) by means of LAN network and TCP/IP protocols.

The firmware of the controller 18 of the receiving unit 3 is set so that the receiving unit 3 can work completely independently from the controller 18. The firmware of the receiving unit 3 carries out cyclically and in sequence the zero-setting and calibration measurements, the temperature measurement and measurement of the signals received from all the elements 7 of the antenna 4.

All the data measured of each single measurement cycle are then transmitted in a single data package with appropriate coding to minimize the dimensions and the load of the LAN network. The coding used and the speed of the LAN network (indicatively 100 Mbps or higher) are more than adequate for transmission of all the data measured with the maximum number of antenna elements scheduled in real time during the measurement.

The low-level operations in the receiving unit 3 are carried out by the hardware devices interfaced with the microprocessor of the receiving unit 3, therefore it is natural and appropriate for these operations to be managed locally by the internal microprocessor.

The operations at a higher hierarchical level are carried out by a code that runs on the embedded operating system (Linux) or by a code that runs on PC/external console. In the second case it is easier to make modifications to the software and therefore, having estimated the impact on the throughput, this second option, not necessary but expedient, was chosen.

In short, the functions implemented on the microprocessor of the receiving unit 3 comprise one or more of the following:

   1. initialization and configuration of the peripherals of the micro;
   2. initialization and configuration of the chips of the receiving unit 3;
   3. management of the procedural parameters that will be read from files; in particular:

   • request for writing/reading of registers at low level;
   • averaging operations at A/D module speed;
   • request for writing/reading of operating parameters;
   • performance of offset zero-setting;
   • request for scanning of a predefined antennae set;
   • request for measurement of frequency only;
   • management of the service functions of the interrupts like timer/counter for frequency measurement;
   • coding of messages received from the probe 8 by modulation of the optical signal in optical fibre 6 (type of keys, state of the probe 8, voltage of the batteries etc.);
- management and signalling of abnormal conditions;

4. cyclic transmission (UDP streaming) of:

- parameters arriving from the probe 8 (status of buttons, battery voltage, status of probe 8, etc.);
- frequency reading;
- receiver chips temperature;
- samples of zero and calibration I, Q for each of the three channels 21
- reading (I1, Q1, I2, Q2, I3, Q3)n repeated for n=1...N; at moment, N=9.

The processing unit 5 comprises:

a) a unit for managing reception via Ethernet of the packages arriving from the receiving unit 3 responsible for:

- receiving the measurement data in UDP streaming and organizing them in an appropriate structure;
- receiving and decoding the value of the timing coming from the buttons of the probe 8.

b) a unit for processing the measurement data in which:

- the calibrations are applied to the raw data;
- module, phase for each channel of each antenna 4 are calculated;
- the actual frequency is calculated starting from the raw value;
- a data structure is constructed containing a time sequence of images coming from the three channels 21 (circular buffer of cluster of arrays 2D)

c) a control and display unit in which:

- a state machine keeps track of the operating context (live / freeze / slow motion / save measurement / ... / error )
- the current image (cluster of arrays 2D) (state: live), the last one acquired before the stop (state: freeze) or the one selected by the operator (state: slow motion) is sent to the display section
- the selected image is saved on disc (suggesting to the operator a context- dependent title, selectable via buttons from a small predefined set).

The measurement phase can be driven remotely by means of four buttons that use the optical fibre transmission channel. This allows the operator to:

- freeze the live image seen on the display
- select (with slow motion effect) the signal pattern deemed the best
- attribute the measurement to one of the physical positions (which will be used for the final statistics)
- save the data thus correlated
- return to measurement mode.

The software is able to display on screen, by means of indication provided by graph, icons and specific text messages, the presence of malfunctions in the system in the event of:

- data line not working/not present/occupied by another application
- out-of-range frequency
- lack of radiofrequency
- battery charge level.

The correct operation of the electromedical system 1 is highlighted by graphic messages and icons.
The main characteristics of the system configuration are therefore one or more of the following:

- transmission by the probe 8 of the test and reference signal for the receiving unit 3 by means of optical fibre 6. This solution limits the possibilities of measurement disturbance by the reference signal transmission connection (for example, via coaxial cable);
- direct locking of the receiving unit 3 onto the signal transmitted by the probe 8. This solution completely eliminates the risk of the receiver locking onto an undesired signal and maximizes rejection of the disturbances from other possible signals present in the RF band. Furthermore, said configuration allows simultaneous measurement of the components in phase and in quadrature (or equivalently amplitude and phase) of the signals received;

- use of an array antenna 4 with switched receiving elements 7. In this way it is possible to measure the field generated by the probe 8 which interacts with the patient's body at different points on a surface positioned at a given distance (antenna surface) and therefore acquire more information to improve the reliability of the diagnosis; and
- use of three receiving channels 21 (and relative filters and diplexer 20) and separate A/D converters with high-speed conversion and transmission of the data to the controller 18. This configuration allows measurements to be taken at high speed and on the three frequencies of the system (fundamental and second and third harmonic) and therefore construction of the field distribution images (amplitude and phase) on the surface of the antenna 4 in real time.

## References

[1] H. Fricke and S. Morse, The Electric Capacity of Tumors of the Breast, J. Cancer Res. 16 (1926) 340.

[2] P.R. C. Gascoyne et al., Dielectrophoretic Separation of Cancer Cells from Blood, IEEE Trans. Ind. Appl. 33 (1997) 670.

[3] S. Sahu et al., Live Visualizations of Single Isolated Tubulin Protein Self-Assembly via Tunneling Current: Effect of Electromagnetic Pumping During Spontaneous Growth of Microtubule, Sci. Rep. 4 (2014) 7303.

[4] See the two movies on:

http://www.nature.com/article-assets/npg/srep/2014/141203/srep07303/etref/srep07303-s3.avi; e
http://www.nature.com/article-assets/npg/srep/2014/141203/srep07303/etref/srep07303-s2.avi

[5] M.L. Martins, A.B. Dinitzen, E. Mamontov, et al. Water dynamics in MCF-7 breast cancer cells: a neutron scattering descriptive study, Sci Rep 9, 8704 (2019).

[6] R.C. Ford, et al. Inelastic Incoherent Neutron Scattering Measurements of Intact Cells and Tissues and Detection of Interfacial Water, J. Am. Chem. Soc. 126, 4682- 4688 (2004).

[7] B. Vinata, Lokeshwar, Diego Rubinowicz, Grethchen, L. Schroeder, E. Forgacsi, J. D. Minna, N. L. Block, and M. Nadji, and B. L. Lokeshwar, Stromal and Epithelial Expression of Tumor Markers Hyaluronic Acid and HYAL1 Hyaluronidase in Prostate Cancer, The Journal of Biological Chemistry, Vol. 276, 11922-11932, 2001.

[8] Y. Kuramoto (1984). Chemical Oscillations, Waves, and Turbulence. New York, NY: Springer-Verlag.

[9] F. Kaiser (1988) Theory of Non-Linear Excitations, in: Fröhlich H. (eds) Biological Coherence and Response to External Stimuli. Springer, Berlin, Heidelberg.
V. Bacot, G. Durey, A. Eddi, M. Fink, E. Fort, Phase-conjugate mirror for water waves driven by the Faraday instability. Proc. Natl. Acad. Sci USA, 2019 Apr 30;116(18):8809-8814. J.C. Cure, On the Electrical Characteristics of Cancer. Second International Congress of Electrochemical Treatment of Cancer, Jupiter (Florida), October 1995.

[10] C.D. Cone, Variation of the Transmembrane Potential Level as a Basic Mecha-nism of Mitosis Control, Oncology 24 (1970) 438.

[11] C.D. Cone, The Role of Surface Electrical Transmembrane Potential in Normal and Malignant Mitogenesis, Ann. N.Y. Acad. Sci. 238 (1975) 420.

[12] F.W. Cope, A Medical Application of the Ling Association-Induction Hypothesis: The High Potassium, Low Sodium Diet of the Gerson Cancer Therapy, Physiol. Chem. Phys. 10 (1978) 465.

## Claims

1. An electromedical system (1) for the non-invasive diagnosis of neoplastic diseases comprising:

   - an electromagnetic source unit (2);
   - a receiving unit (3);
   - a multi-band and multi-channel antenna (4); and
   - a processing unit (5) provided with a data processing software;

   wherein the electromagnetic source unit (2) is configured to generate and radiate an electromagnetic pump signal, an electromagnetic probe signal and a test and reference signal;
   wherein the receiving unit (3) is configured to receive and measure in amplitude and phase;
   the electromagnetic pump signal and the electromagnetic probe signal generated by the electromagnetic source unit (2) and captured, in use, by the multi-band and multi-channel antenna (4), and
   the test and reference signal generated by the electromagnetic source unit (2) and configured to be transmitted to the receiving unit (3) via an optical fibre (6);
   wherein the electromagnetic pump signal is configured to interact with a biological tissue and cause parametric excitation of the biological tissue, which is comparable to a state of Faraday instability;
   wherein the biological tissue behaves like a phase-conjugate mirror when subjected to the electromagnetic probe signal; wherein the electromagnetic probe signal also strikes the biological tissue, thus, giving rise to a phase conjugate signal;
   wherein the electromedical system (1) is configured to analyse the electromagnetic interaction interfering between the test and reference signal and the phase conjugate signal so as to provide information useful for the non-invasive diagnosis of neoplastic diseases;
   wherein the electromagnetic source unit (2) is configured so that the pump signal frequency varies in discrete sub-bands in the range from 300 MHz to 2800 MHz and the frequencies of the electromagnetic probe signal and the test and reference signal are equal to half the pump frequency; and the amplitude of the pump signal lies in the range between 2 V/m and 20 V/m.

**2.** The electromedical system according to claim 1, wherein the electromagnetic source unit (2) comprises harmonic generators to generate the electromagnetic pump signal and the electromagnetic probe signal.

**3.** The electromedical system according to any one of the preceding claims, wherein the electromagnetic source unit (2) comprises a free oscillator configured to generate an RF signal at fundamental frequency, at a UHF band frequency, and harmonics thereof.

**4.** The electromedical system according to any one of the preceding claims, comprising a probe (8) in which the electromagnetic source unit (2) is contained; wherein the probe (8) is configured so as to be hand-held and used for scanning an area under examination.

**5.** The electromedical system according to claim **4,** wherein the receiving unit (3) comprises three receiving channels to simultaneously receive a signal transmitted by the probe (8) at a fundamental frequency and at a second and a third harmonic.

**6.** The system according to claim **4** or **5,** wherein the probe (8) also comprises power-supply and control circuits and a high-speed fibre optic transmitter (19) which draws a part of the signal of an oscillator of the electromagnetic source unit (2) and transmits it in the optical fibre (6).

**7.** The system according to any one of the preceding claims, wherein the receiving unit (3) is configured to receive signals and correlating them in space and time.

**8.** The system according to any one of the preceding claims, wherein the multi-band and multi-channel antenna (4) is configured to operate as a spatial and spectral filter by means of its radiating configuration of an array of independent and decoupled elements (7) and distributed in space for mapping the distribution of the signal received.

**9.** The system according to any one of the preceding claims, wherein the antenna (4) comprises dipole elements (7) configured to receive in broadband and to allow measurement of the electromagnetic field generated by the probe (8) and which interacts with a district or an organ of the patient under examination at different points on a surface positioned at a given distance.

**10.** The system according to claim **9,** wherein the dipole elements (7) are passive broadband elements or elements resonating at one of the frequencies received or active elements.

**11.** The electromedical system according to claim **9** or **10,** further comprising a high-insulation radiofrequency switching device (19) configured to allow the signal received by each dipole element (7) to be sent to the receiving unit (3).

**Patentansprüche**

**1.** Elektromedizinisches System (1) für die nichtinvasive Diagnose von neoplastischen Erkrankungen, umfassend:

- eine elektromagnetische Quelleneinheit (2);
- eine Empfangseinheit (3);
- eine Mehrband- und Mehrkanalantenne (4) und
- eine Verarbeitungseinheit (5), die mit einer Datenverarbeitungssoftware versehen ist,

wobei die elektromagnetische Quelleneinheit (2) ausgelegt ist, um ein elektromagnetisches Pumpsignal, ein elektromagnetisches Abtastsignal und ein Test- und Referenzsignal zu erzeugen und abzustrahlen, wobei die Empfangseinheit (3) für den Empfang sowie die amplituden- und phasenmäßige Messung ausgelegt ist,

wobei das elektromagnetische Pumpsignal und das elektromagnetische Abtastsignal von der elektromagnetischen Quelleneinheit (2) erzeugt und bei Gebrauch von der Mehrband- und Mehrkanalantenne (4) erfasst werden und

das Test- und Referenzsignal von der elektromagnetischen Quelleneinheit (2) erzeugt wird und ausgelegt ist, um über einen Lichtwellenleiter (6) an die Empfangseinheit (3) übertragen zu werden,

wobei das elektromagnetische Pumpsignal ausgelegt ist, um mit einem biologischen Gewebe zu interagieren und eine parametrische Anregung des biologischen Gewebes herbeizuführen, die mit einem Zustand der Faraday-Instabilität vergleichbar ist,

wobei sich das biologische Gewebe bei Beaufschlagung mit dem elektromagnetischen Abtastsignal wie ein phasenkonjugierender Spiegel verhält, wobei das elektromagnetische Abtastsignal ebenfalls auf das biologische Gewebe trifft und dadurch die Entstehung eines phasenkonjugierten Signals bewirkt,

wobei das elektromedizinische System (1) ausgelegt ist, um die elektromagnetische Interaktion zu analysieren, die zwischen dem Test- und Referenzsignal und dem phasenkonjugierten Signal interferiert, sodass nützliche Informationen für die nichtinvasive Diagnose neoplastischer Erkrankungen bereitgestellt werden, wobei die elektromagnetische Quelleneinheit (2) derart ausgelegt ist, dass die Pumpsignalfrequenz in voneinander getrennten Subbändern im Bereich von 300 MHz bis 2800 MHz variiert und die Frequenzen des elektromagnetischen Abtastsignals sowie des Test- und Referenzsignals jeweils gleich der halben Pumpfrequenz sind, und wobei die Amplitude des Pumpsignals im Bereich zwischen 2 V/m und 20 V/m liegt.

2. Elektromedizinisches System nach Anspruch 1, wobei die elektromagnetische Quelleneinheit (2) Frequenzvervielfacher umfasst, um das elektromagnetische Pumpsignal und das elektromagnetische Abtastsignal zu erzeugen.

3. Elektromedizinisches System nach einem der vorhergehenden Ansprüche, wobei die elektromagnetische Quelleneinheit (2) einen freien Oszillator umfasst, der ausgelegt ist, um ein HF-Signal mit einer Grundfrequenz, einer UHF-Bandfrequenz und deren Harmonischen zu erzeugen.

4. Elektromedizinisches System nach einem der vorhergehenden Ansprüche, umfassend eine Sonde (8), in der die elektromagnetische Quelleneinheit (2) enthalten ist, wobei die Sonde (8) ausgelegt ist, um in der Hand gehalten zu werden, und genutzt wird, um einen untersuchten Bereich abzutasten.

5. Elektromedizinisches System nach Anspruch 4, wobei die Empfangseinheit (3) drei Empfangskanäle umfasst, um gleichzeitig ein von der Sonde (8) übertragenes Signal mit einer Grundfrequenz sowie mit einer zweiten und einer dritten Harmonischen zu empfangen.

6. System nach Anspruch 4 oder 5, wobei die Sonde (8) ferner Stromversorgungs- und Steuerschaltungen sowie einen Hochgeschwindigkeits-Lichtwellenleiter-Sender (19) umfasst, der einen Teil des Signals eines Oszillators der elektromagnetischen Quelleneinheit (2) abgreift und über den Lichtwellenleiter (6) überträgt.

7. System nach einem der vorhergehenden Ansprüche, wobei die Empfangseinheit (3) ausgelegt ist, um Signale zu empfangen und diese räumlich und zeitlich zu korrelieren.

8. System nach einem der vorhergehenden Ansprüche, wobei die Mehrband- und Mehrkanalantenne (4) ausgelegt ist, um mittels ihrer abstrahlenden Auslegung eines Arrays aus unabhängigen und entkoppelten Elementen (7), die räumlich verteilt sind, um die Verteilung des empfangenen Signals abzubilden, als räumlicher und spektraler Filter zu arbeiten.

9. System nach einem der vorhergehenden Ansprüche, wobei die Antenne (4) Dipolelemente (7) umfasst, die ausgelegt sind, um das von der Sonde (8) erzeugte elektromagnetische Feld, das mit einem Bereich oder einem Organ des untersuchten Patienten an verschiedenen Positionen auf einer in einem vorgegebenen Abstand positionierten Oberfläche interagiert, breitbandig zu empfangen und dessen Messung zu ermöglichen.

10. System nach Anspruch 9, wobei die Dipolelemente (7) passive breitbandige Elemente oder bei einer der empfangenen Frequenzen resonierende Elemente oder aktive Elemente sind.

11. Elektromedizinisches System nach Anspruch 9 oder 10, ferner umfassend eine hochisolierende Hochfrequenz-Umschaltvorrichtung (19), die so ausgelegt ist, dass das von jedem Dipolelement (7) empfangene Signal an die Empfangseinheit (3) gesendet werden kann.

**Revendications**

1. Système électromédical (1) pour le diagnostic non invasif de maladies néoplasiques comprenant :

- une unité de source électromagnétique (2) ;
- une unité de réception (3) ;
- une antenne multibande et multicanal (4) ; et
- une unité de traitement (5) dotée d'un logiciel de traitement de données ;
dans lequel l'unité de source électromagnétique (2) est configurée pour générer et émettre un signal de pompe électromagnétique, un signal de sonde électromagnétique et un signal de test et de référence ; dans lequel l'unité de réception (3) est configurée pour recevoir et mesurer en amplitude et en phase ;
le signal de pompe électromagnétique et le signal de sonde électromagnétique étant générés par l'unité de source électromagnétique (2) et captés, en cours d'utilisation, par l'antenne multibande et multicanal (4), et
le signal d'essai et de référence étant généré par l'unité de source électromagnétique (2) et configuré pour être transmis à l'unité de réception (3) par l'intermédiaire d'une fibre optique (6) ;
dans lequel le signal de la pompe électromagnétique est configuré pour interagir avec un tissu biologique et provoquer une excitation paramétrique du tissu biologique, qui est comparable à un état d'instabilité de Faraday ;
dans lequel le tissu biologique se comporte comme un miroir à conjugaison de phase lorsqu'il est soumis au signal de sonde électromagnétique ;

dans lequel le signal de sonde électromagnétique frappe également le tissu biologique, donnant ainsi lieu à un signal à conjugaison de phase ;

dans lequel le système électromédical (1) est configuré pour analyser l'interaction électromagnétique interférant entre le signal de test et de référence et le signal à conjugaison de phase afin de fournir des informations utiles pour le diagnostic non invasif des maladies néoplasiques ;

dans lequel l'unité de source électromagnétique (2) est configurée de telle sorte que la fréquence de signal de pompe varie dans des sous-bandes discrètes comprises entre 300 MHz et 2800 MHz et que les fréquences du signal de sonde électromagnétique et du signal de test et de référence soient égales à la moitié de la fréquence de pompe ; et l'amplitude du signal de pompe est comprise entre 2 V/m et 20 V/m.

2. Système électromédical selon la revendication 1, dans lequel l'unité de source électromagnétique (2) comprend des générateurs d'harmoniques pour générer le signal de pompe électromagnétique et le signal de sonde électromagnétique.

3. Système électromédical selon l'une quelconque des revendications précédentes, dans lequel l'unité de source électromagnétique (2) comprend un oscillateur libre configuré pour générer un signal RF à la fréquence fondamentale, à une fréquence de la bande UHF, et à leurs harmoniques.

4. Système électromédical selon l'une quelconque des revendications précédentes, comprenant une sonde (8) dans laquelle est contenue l'unité de source électromagnétique (2) ; dans lequel la sonde (8) est configurée de manière à pouvoir être tenue à la main et utilisée pour balayer une zone en cours d'examen.

5. Système électromédical selon la revendication 4, dans lequel l'unité de réception (3) comprend trois canaux de réception pour recevoir simultanément un signal transmis par la sonde (8) à une fréquence fondamentale et à une deuxième et une troisième harmonique.

6. Système selon la revendication 4 ou 5, dans lequel la sonde (8) comprend également des circuits d'alimentation électrique et de commande et un émetteur à fibre optique à grande vitesse (19) qui extrait le signal d'un oscillateur de l'unité de source électromagnétique (2) et le transmet dans la fibre optique (6).

7. Système selon l'une quelconque des revendications précédentes, dans lequel l'unité de réception (3) est configurée pour recevoir des signaux et les corréler dans l'espace et le temps.

8. Système selon l'une quelconque des revendications précédentes, dans lequel l'antenne multibande et multicanal (4) est configurée pour fonctionner comme un filtre spatial et spectral au moyen de sa configuration de rayonnement d'un réseau d'éléments indépendants et découplés (7) et distribués dans l'espace pour cartographier la distribution du signal reçu.

9. Système selon l'une quelconque des revendications précédentes, dans lequel l'antenne (4) comprend des éléments dipolaires (7) configurés pour recevoir en large bande et permettre la mesure du champ électromagnétique généré par la sonde (8) et qui interagit avec un quartier ou un organe du patient en cours d'examen en différents points sur une surface positionnée à une distance donnée.

10. Système selon la revendication 9, dans lequel les éléments dipolaires (7) sont des éléments passifs à large bande ou des éléments résonnant à l'une des fréquences reçues ou des éléments actifs.

11. Système électromédical selon la revendication 9 ou 10, comprenant en outre un dispositif de commutation radiofréquence à haute isolation (19) configuré pour permettre au signal reçu par chaque élément dipôle (7) d'être envoyé à l'unité de réception (3).

FIG. 1

FIG. 2

FIG. 3

$$E(x,y,z,t) = A(x,y) \left| e^{j[kz+\phi(x,y)]-\omega t} + e^{-j[kz+\phi(x,y)]-\omega t} \right|$$

$$E_c(x,y,z,t) = A(x,y) \left| e^{j[kz+\phi(x,y)]+\omega t} + e^{-j[kz+\phi(x,y)]+\omega t} \right|$$

FIG. 4

FIG. 5

Pump
signal

Test and
reference signal

Biological tissue

Probe
signal

FIG. 6

Pump
signal

Biological tissue

Case of a
healthy
patient

Pump
signal

Biological tissue

Parametric
instability

Case of
a sick
patient

FIG. 7

ALPHA

FIG. 8

ALPHA

FIG. 9

FIG. 10

FIG. 11

EP 4 266 985 B1

FIG. 12

FIG. 13

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2008171949 A

**Non-patent literature cited in the description**

- **MARGHERITA et al.** *JACS*, 2020, vol. 142, 3-15
- Non invasive radiofrequency diagnostics of cancer. The Bioscanner Trimprob technology and clinical applications. **CLARBRUNO VEDRUCCIO et al.** JOURNAL OF PHYSICS: CONFERENCE SERIES. INSTITUTE OF PHYSICS PUBLISHING, 13 December 2011, vol. 329
- **H. FRICKE ; S. MORSE**. The Electric Capacity of Tumors of the Breast,. *J. Cancer Res.*, 1926, vol. 16, 340
- **P.R. C. GASCOYNE et al.** Dielectrophoretic Separation of Cancer Cells from Blood. *IEEE Trans. Ind. Appl.*, 1997, vol. 33, 670
- **S. SAHU et al.** Live Visualizations of Single Isolated Tubulin Protein Self-Assembly via Tunneling Current: Effect of Electromagnetic Pumping During Spontaneous Growth of Microtubule. *Sci. Rep.*, 2014, vol. 4, 7303
- **M.L. MARTINS ; A.B. DINITZEN ; E. MAMONTOV et al.** Water dynamics in MCF-7 breast cancer cells: a neutron scattering descriptive study. *Sci Rep*, 2019, vol. 9, 8704
- **R.C. FORD et al.** Inelastic Incoherent Neutron Scattering Measurements of Intact Cells and Tissues and Detection of Interfacial Water. *J. Am. Chem. Soc.*, 2004, vol. 126, 4682-4688
- **B. VINATA ; LOKESHWAR, DIEGO ; RUBINOWICZ, GRETHCHEN ; L. SCHROEDER ; E. FORGACSI ; J. D. MINNA ; N. L. BLOCK ; M. NADJI ; B. L. LOKESHWAR**. Stromal and Epithelial Expression of Tumor Markers Hyaluronic Acid and HYAL1 Hyaluronidase in Prostate Cancer. *The Journal of Biological Chemistry*, 2001, vol. 276, 11922-11932
- **Y. KURAMOTO**. Chemical Oscillations, Waves, and Turbulence.. Springer-Verlag., 1984
- Theory of Non-Linear Excitations. **F. KAISER**. Biological Coherence and Response to External Stimuli.. Springer, 1988
- **V. BACOT ; G. DUREY ; A. EDDI ; M. FINK ; E. FORT**. Phase-conjugate mirror for water waves driven by the Faraday instability.. *Proc. Natl. Acad. Sci USA*, 30 April 2019, vol. 116 (18), 8809-8814
- **J.C. CURE**. On the Electrical Characteristics of Cancer.. *Second International Congress of Electrochemical Treatment of Cancer*, October 1995
- **C.D. CONE**. Variation of the Transmembrane Potential Level as a Basic Mecha-nism of Mitosis Control. *Oncology*, 1970, vol. 24, 438
- **C.D. CONE**. The Role of Surface Electrical Transmembrane Potential in Normal and Malignant Mitogenesis. *Ann. N.Y. Acad. Sci.*, 1975, vol. 238, 420
- **F.W. COPE**. A Medical Application of the Ling Association-Induction Hypothesis: The High Potassium, Low Sodium Diet of the Gerson Cancer Therapy. *Physiol. Chem. Phys.*, 1978, vol. 10, 465